# EUROPEAN PATENT APPLICATION

(11) **EP 3 447 470 A1**
(43) Date of publication of application: **27.02.2019**
(21) Application number: 18186769.8
(22) Date of filing: 01.08.2018
(51) Int. Cl.: G01N 1/22, G01N 33/00, H01L 41/053, H01L 41/09, H04M 1/02

(54) **ACTUATING AND SENSING APPARATUS AND CASE USING THE SAME**

(30) Priority: 21.08.2017 TW 10628276
(71) Applicant: Microjet Technology Co., Ltd, Hsinchu (TW)
(72) Inventor: Mou, Hao-Jan, Hsinchu (TW); Liao, Jia-Yu, Hsinchu (TW); Chen, Shih-Chang, Hsinchu (TW); Chen, Shou-Hung, Hsinchu (TW); Chen, Mei-Yen, Hsinchu (TW); Huang, Chi-Feng, Hsinchu (TW); Lee, Wei-Ming, Hsinchu (TW)
(74) Representative: Uexküll & Stolberg

(57) **Abstract**

An actuating and sensing apparatus (1) includes a circuit board (10), a housing (11), an actuating device (12) and a sensor (13). The housing (11) is disposed on the circuit board (10) and has an inhale aperture (111a), a discharge aperture (111b) and a compartment (115). The compartment (115) is in communication with the exterior of the housing (11) through the inhale aperture (111a) and the discharge aperture (111b). The actuating device (12) is disposed within the compartment (115). The sensor (13) is disposed within the compartment (115) and corresponding in position to the inhale aperture (111a). When the actuating device (12) is driven, gas within the compartment (115) is guided to the exterior of the housing (11), and a pressure gradient is generated in the compartment (115) so as to introduce the gas from the exterior of the housing (11) into the compartment (115) through the inhale aperture (111a) to make the gas measured by the sensor (13).

## Description

### FIELD OF THE INVENTION

The present disclosure relates to an actuating and sensing apparatus, and more particularly to an actuating and sensing apparatus and a case using the same, which transport gas by an actuating device, and the gas is inhaled and discharged through the same side wall of the actuating and sensing apparatus and the case using the same.

### BACKGROUND OF THE INVENTION

Nowadays, people pay much attention to the air quality in the environment. For example, it has become common to monitor air pollutants in the environment, such as carbon monoxide, carbon dioxide, volatile organic compounds (VOC) and PM2.5, since the exposure of these substances can cause human health problems or even can be life-threatening. Therefore, environmental air monitoring has been taken seriously by most countries and has become a considerable issue in our daily life.

However, the conventional environmental gas measuring device (e.g., the air cleaner with air quality measure function) is bulky in volume and not easily to be carried. As a result, it is difficult for a user to acquire information relating to the environmental gas accurately. In other words, the user is highly possible to be exposed to the toxic environment. Therefore, it is important to solve the problem that how the user can acquire the information relating to the environmental gas in everywhere and at any time.

Besides, the conventional environmental gas measuring device generally has no waterproof and dustproof function. Under this circumstance, if moisture or liquid enter the environmental gas measuring device during gas circulation, the gas flow would contain moisture and make the electronic component (e.g., gas sensor) damped, rusted or even damaged. Moreover, if dust or particles enter the environmental gas measuring device, the electronic components would be damaged and the gas transportation efficiency would be reduced. Therefore, it is also important to achieve the waterproof and dustproof efficacy of the environmental gas measuring device.

For solving the above drawbacks, it is important to provide a miniature, silent, waterproof and dustproof actuating and sensing apparatus for using in a portable electronic device so as to achieve the portable purpose and the air quality can be immediately monitored everywhere and at any time.

### SUMMARY OF THE INVENTION

An object of the present disclosure provides an actuating and sensing apparatus by which the gas is inhaled and discharged through the same side wall. When a piezoelectric actuator of the actuating and sensing apparatus is activated, a pressure gradient is generated in the fluid channel to facilitate the gas to flow at a high speed. Moreover, since there is an impedance difference between the inhaling direction and the discharging direction of the fluid channel, the gas is transported from the inhale end to the discharge end, which are at the same side wall. Consequently, the problem existing in the prior art that the volume of equipment or a machine with gas transportation function is bulky, noisy and not portable, is solved. In addition, due to the air circulating design that the gas is inhaled and discharged through the same side wall, the actuating and sensing apparatus could be widely applied to the portable electronic device.

Another object of the present disclosure provides an actuating and sensing apparatus having waterproof and dustproof efficacy. The portable electronic device is equipped with at least one protective film to filter out the moisture and the dust. Consequently, the problem existing in the prior art that the moisture and the dust enter the inside of the device to ruin the components therein and decrease gas transportation efficiency, is solved.

A further object of the present disclosure provides a case of an electronic device mounted with an actuating and sensing apparatus. Since the actuating and sensing apparatus is miniature and silent, and the gas is inhaled and discharged through the same side wall, it may be installed in the casing of a smart phone, thereby the problem existing in the prior art that the conventional environmental gas measuring device is inconvenient to carry and the user is unable to acquire information relating to the environmental gas in everywhere and at any time, is solved.

In accordance with an aspect of the present disclosure, an actuating and sensing apparatus is provided. The actuating and sensing apparatus includes a circuit board, a housing, an actuating device and a sensor. The housing is disposed on the circuit board and has a compartment. The compartment is in communication with the exterior of the housing through an inhale aperture and a discharge aperture disposed on a first side wall. The actuating device and the sensor are disposed within the compartment. When the actuating device is driven, the gas within the compartment is guided to the exterior of the housing through the discharge aperture, and a pressure gradient is generated in the compartment so as to introduce the gas from the exterior of the housing into the compartment through the inhale aperture to make the gas measured by the sensor.

In accordance with another aspect of the present disclosure, a case for using in a portable electronic device is provided. The case includes an actuating and sensing apparatus, a plate and a lateral wall. The actuating and sensing apparatus includes a circuit board, a housing, an actuating device and a sensor. The housing is disposed on the circuit board and has a compartment. The compartment is in communication with the exterior of the housing through an inhale aperture and a discharge aperture disposed on a first side wall. The actuating device and the sensor are disposed within the compartment. The lateral wall extends from the plate and has a first wall section. An inlet and an outlet are disposed on the first wall section. The actuating and sensing apparatus are disposed on the plate. The inhale aperture and the discharge aperture of the actuating and sensing apparatus are disposed corresponding in position to the inlet and the outlet of the first wall section, respectively. When the actuating device of the actuating and sensing apparatus is driven, gas within the compartment is guided to the exterior of the case through the discharge aperture and the outlet in sequence, and a pressure gradient is generated in the compartment so as to introduce the gas from the exterior of the case into the compartment through the inlet and the inhale aperture in sequence, to make the gas measured by the sensor.

The above contents of the present disclosure will become more readily apparent to those ordinarily skilled in the art after reviewing the following detailed description and accompanying drawings, in which:

### BRIEF DESCRIPTION OF THE DRAWINGS

FIG. 1A is a schematic perspective view illustrating an actuating and sensing apparatus according to some embodiments of the present disclosure;
FIG. 1B is another schematic perspective view illustrating the actuating and sensing apparatus according to some embodiments of the present disclosure;
FIG. 1C is a schematic perspective view illustrating a circuit board and a sensor of the actuating and sensing apparatus;
FIG. ID is a schematic perspective view illustrating an actuating device and a housing of the actuating and sensing apparatus;
FIG. 2 is a schematic cross-sectional view illustrating the actuating and sensing apparatus according to some embodiments of the present disclosure;
FIG. 3A is a schematic exploded view illustrating the actuating device of the actuating and sensing apparatus;
FIG. 3B is another schematic exploded view illustrating the actuating device of the actuating and sensing apparatus;
FIG. 4A is a schematic perspective view illustrating a piezoelectric actuator of the actuating device of the actuating and sensing apparatus;
FIG. 4B is another schematic perspective view illustrating the piezoelectric actuator of the actuating device of the actuating and sensing apparatus;
FIG. 4C is a schematic cross-sectional view illustrating the piezoelectric actuator of the actuating device of the actuating and sensing apparatus;
FIGS. 5A to 5E are schematic diagrams illustrating the actuations of the actuating device of the actuating and sensing apparatus;
FIG. 6A is a schematic perspective view illustrating the actuating and sensing apparatus and a case of an electronic device according to some embodiments of the present disclosure;
FIG. 6B is a partially enlarged schematic view of the actuating and sensing apparatus and the case of the electronic device according to some embodiments of the present disclosure; and
FIG. 6C is another schematic perspective view illustrating the case of the electronic device according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

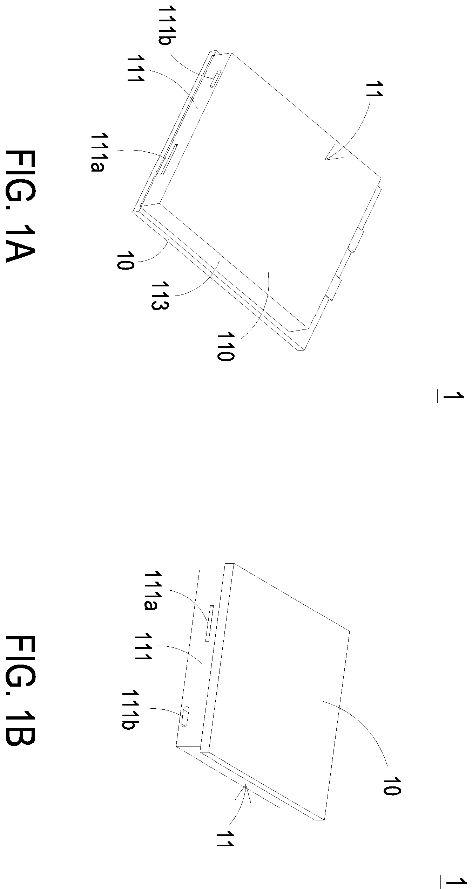

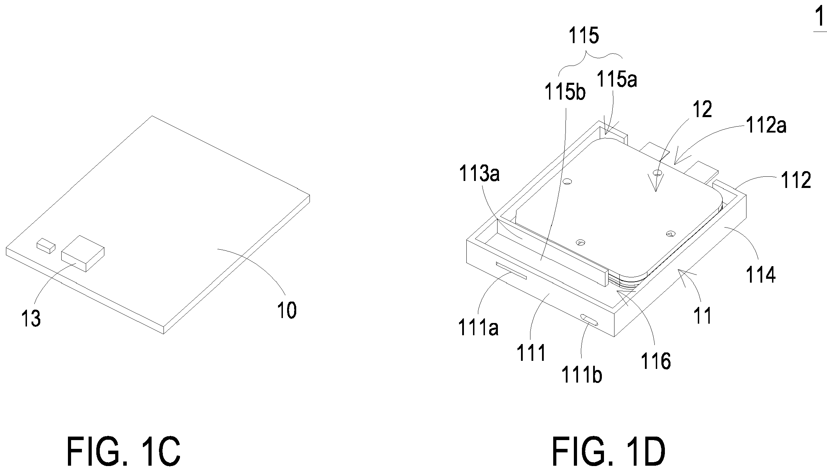

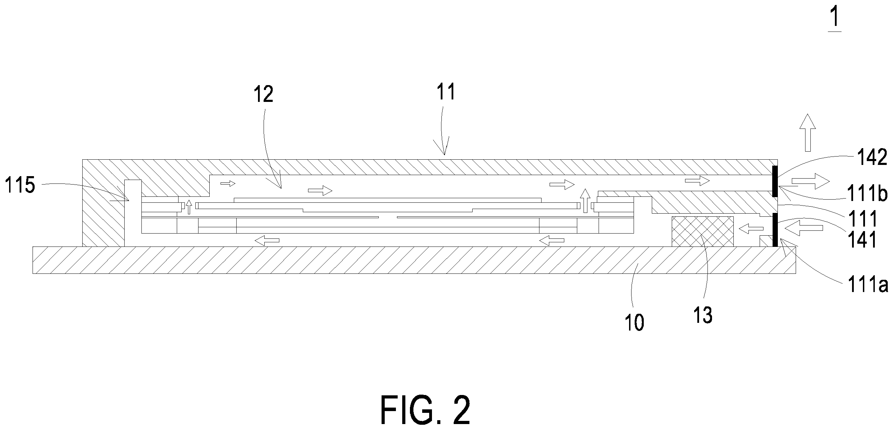

FIG. 1A is a schematic perspective view illustrating an actuating and sensing apparatus according to some embodiments of the present disclosure. FIG. 1B is another schematic perspective view illustrating the actuating and sensing apparatus according to some embodiments of the present disclosure. FIG. 1C is a schematic perspective view illustrating a circuit board 10 and a sensor of the actuating and sensing apparatus. FIG. 2 is a schematic cross-sectional view illustrating the actuating and sensing apparatus according to some embodiments of the present disclosure. Referring to FIGS. 1A to 2, the present discourse provides an actuating and sensing apparatus 1 including at least one circuit board 10, at least one housing 11 having at least one compartment 115, at least one inhale aperture 111a and at least one discharge aperture 111b, at least one actuating device 12 and at least one sensor 13. The number of the circuit board 10, the housing 11, the compartment 115, the inhale aperture 111a, the discharge aperture 111b, the actuating device 12 and the sensor 13 is exemplified by one for each in the following embodiments but not limited thereto. It is noted that each of the circuit board 10, the housing 11, the compartment 115, the inhale aperture 111a, the discharge aperture 111b, the actuating device 12 and the sensor 13 can also be provided in plural numbers.

The actuating and sensing apparatus 1 of the present disclosure is disposed for measuring the quality of the environmental gas while achieving the waterproof, dustproof and silent efficacy. The actuating and sensing apparatus 1 could be used in any portable electronic device. For example, the portable electronic devices may be a notebook, a smart phone, a smart band, a tablet or any other appropriate portable electronic device. In some embodiments, the actuating and sensing apparatus 1 includes the circuit board 10, the housing 11, the actuating device 12 and the sensor 13. In some embodiments, the circuit board 10 is a platform for integrating the housing 11, the actuating device 12 and the sensor 13. The circuit board 11 could be but not limited to a printed circuit board (PCB) for installing the housing 11, the actuating device 12 and the sensor 13, and providing driving power to the actuating device 12 and the sensor 13, thereby to enable the actuating device 12 and the sensor 13. As shown in FIGS. 1A and ID, the housing 11 is a square shell structure, and has composed of a housing wall 110 and four side walls 111, 112, 113 and 114. The first side wall 111 is disposed opposite to the second side wall 112, and the third side wall 113 is disposed opposite to the fourth side wall 114. The compartment 115 is defined by the four side walls 111, 112, 113, 114 and the housing wall 110, and is disposed for receiving the actuating device 12 and the sensor 13. As shown in FIG. 1A, the housing 11 is disposed on the circuit board 10. The inhale aperture 111a and the discharge aperture 111b are formed in the first side wall 111 of the housing 11 and are disposed for inhaling gas from the inhale aperture 111a into the compartment 115 and discharging the gas through the discharge aperture 111b. As shown in FIG. ID, in some embodiments, the second side wall 112 is formed with a pin opening 112a. In some other embodiments, the pin opening 112a may be omitted. In some embodiments, the housing 11 further has a partition 113a disposed in the compartment 115 and extending perpendicularly from the third side wall 113. The partition 113a and the fourth side wall 114 collaboratively define an opening 116. The compartment 115 is divided into a first chamber 115a and a second chamber 115b by the partition 113a. The first chamber 115a is in communication with the second chamber 115b through the opening 116, so that the gas could flow therethrough. The second chamber 115b could be in communication with the exterior of the housing 11 through the inhale aperture 111a and the discharge aperture 111b of the first side wall 111. In some other embodiments, the housing wall 110, the four side walls 111, 112, 113, 114 and the partition 113a are formed as one piece. In some other embodiments, such configuration may be varied. Moreover, the housing 11 may be made of thermosetting plastic material, but no limited thereto.

Referring to FIGS. 1C and ID. in some embodiments, the actuating device 12 is disposed within the first chamber 115a, and the second chamber 115b is configured as an air flow channel. In some embodiments, the sensor 13 is disposed on the circuit board 10, is disposed in the second chamber 115b, and is aligned with the inhale aperture 111a of the first side wall 111 of the housing 11 for measuring the concentrations and contents of certain substances in the air. In some other embodiments, the sensor 13 may be aligned with the discharge aperture 111b. It is noted that, the disposition of the sensor 13 is not limited to the above embodiments, and is adjustable according the practical requirements.

Referring to FIGS. 1A, ID and 2, when the actuating device 12 is driven, a pressure gradient is generated in the compartment 115 so as to introduce the gas from the exterior of the housing 11 into the compartment 115 through the inhale aperture 111a to make the gas measured by the sensor 13. Consequently, information relating to environmental gas is acquired. Moreover, the gas within the compartment 115 is discharged to the exterior of the housing 11 through the discharge aperture 111b of the first side wall 111, so that an air circulation is formed by the gas inhaled and discharged through the first side wall 111. As the actuating device 12 is continuously driven, the gas is continuously guided into the compartment 115, and the gas flows through the inhale aperture 111a, the compartment 115, the sensor 13 and the actuating device 12 in sequence, and is then discharged through the discharge aperture 111b. Since the concentrations and contents of the gas in the compartment 115 are continuously measured by the sensor 13, the information relating to the environmental gas can be acquired in real time. In some embodiments, the speed of the gas circulation is accelerated by the actuator 12, so that the sensor 13 can measure the information of environmental gas immediately. When the environmental gas is measured to be toxic or dangerous, a user could be reminded to take protective actions immediately for preventing gas poisoning and gas explosion.

In some embodiments, the sensor 13 is configured as but not limited to at least one selected from the group consisting an oxygen sensor, a carbon monoxide sensor, a carbon dioxide sensor, a temperature sensor, an ozone sensor, a particulate sensor (e.g., a PM2.5 particle sensor), a sulfur dioxide sensor, a nitrogen dioxide sensor, a volatile organic compound sensor (e.g., a sensor for measuring formaldehyde or ammonia nitrogen), and a combination thereof.

Further referring to FIG. 2, the actuating and sensing apparatus 1 further includes a first protective film 141 and a second protective film 142. The first protective film 141 and the second protective film 142 are respectively and closely disposed at the inhale aperture 111a and the discharge aperture 111b. The first protective film 141 and the second protective film 142 are waterproof and dustproof film structures, which allow the gas to pass therethrough. The disposition of the first protective film 141 and the second protective film 142 prevents the moisture and the dust from entering the compartment 115 through the inhale aperture 111a and the discharge aperture 111b, such that the actuating device 12 and the sensor 13 within the compartment 115 are prevented from being rusted or damaged. In some embodiments, the first protective film 141 and the second protective film 142 comply with IP64 waterproof and dustproof rating of International Protection Marking (IEC 60529), i.e., the dust protection level is 6 (Complete protection, No ingress of dust) and the water protection level is 4 (Protection against splash: Water splashing to equipment from any direction shall have no negative effects). In some other embodiments, the first protective film 141 and the second protective film 142 comply with IP68 waterproof and dustproof rating of International Protection Marking (IEC 60529), i.e., the dust protection level is 6 and the water protection level is 8 (Continuous immersion in the water shall have no negative effects).

In some embodiments, the actuating and sensing apparatus 1 further includes a battery (not shown). The battery is disposed within the housing 11 and electrically connected to the circuit board 10. That is, the battery of the actuating and sensing apparatus 1 is directly used as the power source for generating the driving power. The driving power is transported to the actuating device 12 and the sensor 13 through the circuit board 10. In some other embodiments, the circuit board 10 is electrically connected to an external power source (not shown). For example, a charger, a rechargeable battery, or a wireless rechargeable component, which is not restricted.

In some embodiments, the actuating device 12 is configured as an air pump having a resonance-type piezoelectric actuator, but not limited thereto. In some other embodiments, the actuating device 12 may be a motor or a pump having one selected from the group consisting of an electric actuating device, a magnetic actuating device, a thermal actuating device, a piezoelectric actuating device and a fluid actuating device. For example, the actuating device 12 may be but not limited to a DC/AC/step motor with the electric actuating device, a magnetic coil motor with the magnetic actuating device, a heat pump with the thermal actuating device, or an air/liquid pump with the fluid actuating device.

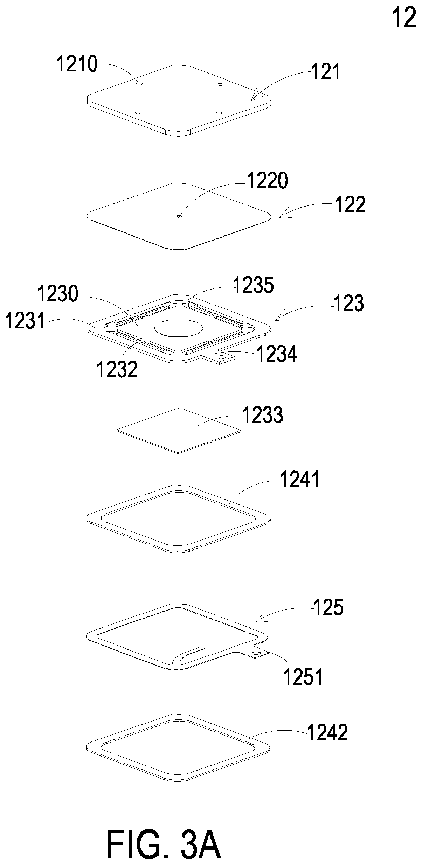

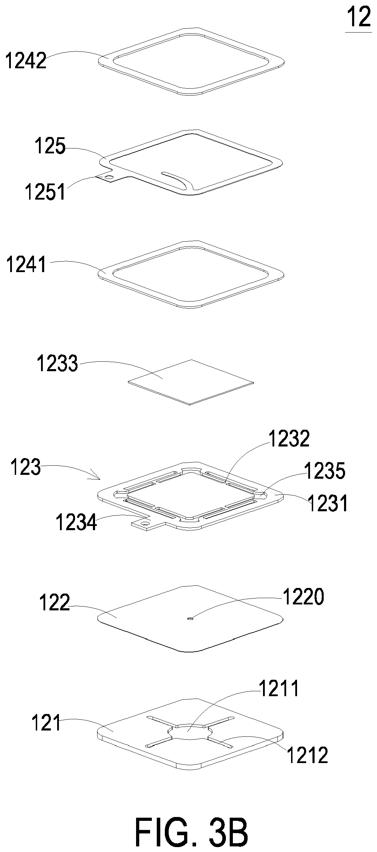

FIG. 3A is a schematic exploded view illustrating the actuating device of the actuating and sensing apparatus. FIG. 3B is another schematic exploded view illustrating the actuating device of the actuating and sensing apparatus. Referring to FIGS. 3A and 3B, the actuating device 12 includes a gas inlet plate 121, a resonance plate 122, a piezoelectric actuator 123, a first insulation plate 1241, a conducting plate 125 and a second insulation plate 1242, which are stacked on each other sequentially. The piezoelectric actuator 123 includes a suspension plate 1230 and a piezoelectric plate 1233, and is aligned with the resonance plate 122. The gas inlet plate 121 has at least one gas inlet hole 1210 formed in a surface of the gas inlet plate 121. The gas is inhaled into the actuating device 12 through at least one gas inlet hole 1210 of the gas inlet plate 121, and flows through a plurality of chambers (not shown), so as to transport the gas.

As shown in FIG. 3A, in some embodiments, the gas inlet plate 121 has four gas inlet holes 1210. It is noted that, the number of the at least one gas inlet hole 1210 is not restricted. As shown in FIG. 3B, the gas inlet plate 121 further has a central cavity 1211 and at least one convergence channel 1212 formed in an opposite surface of the gas inlet plate 121. The at least one convergence channel 1212 is aligned with the at least one gas inlet hole 1210. In some embodiments, the gas inlet plate 121 has four convergence channels 1212 corresponding in position to the four gas inlet holes 1210. The gas inhaled from the at least one gas inlet hole 1210 is guided through the at least one convergence channel 1212 to the central cavity 1211 and is then further transported. In some embodiments, the at least one gas inlet hole 1210, the at least one convergence channel 1212 and the central cavity 1211 of the gas inlet plate 121 are formed as one piece. The central cavity 1211 is configured as a convergence chamber for temporarily storing the gas. In some embodiments, the gas inlet plate 121 is, for example, made of stainless steel, but no limit thereto. In some other embodiments, the depth of the convergence chamber defined by the central cavity 1211 is equal to the depth of the at least one convergence channel 1212, but no limit thereto.

In some embodiment, the resonance plate 122 is made of flexible material, but no limit thereto. The resonance plate 122 has a central aperture 1220 formed corresponding in position to the central cavity 1211 of the gas inlet plate 121, such that the central aperture 1220 allows the gas to be transferred therethrough. In some other embodiments, the resonance plate 122 is, for example, made of copper, but no limit thereto.

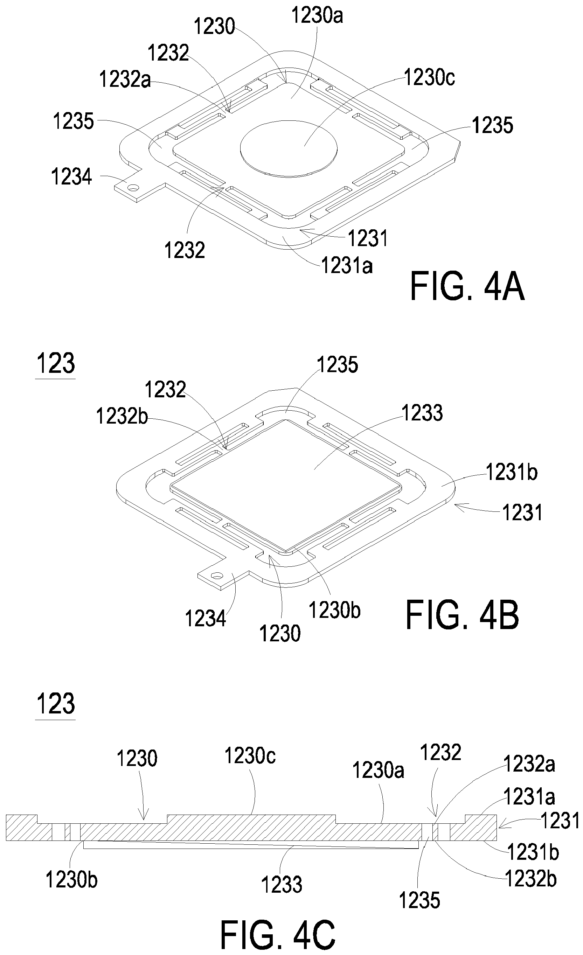

FIG. 4A is a schematic perspective view illustrating the piezoelectric actuator of the actuating device of the actuating and sensing apparatus. FIG. 4B is another schematic perspective view illustrating the piezoelectric actuator of the actuating device of the actuating and sensing apparatus. FIG. 4C is a schematic cross-sectional view illustrating the piezoelectric actuator of the actuating device of the actuating and sensing apparatus. Referring to FIGS. 4A to 4C, in some embodiments, the piezoelectric actuator 123 further includes an outer frame 1231 and a plurality of brackets 1232. The suspension plate 1230 has a first surface 1230a and a second surface 1230b. The piezoelectric plate 1233 is attached on the second surface 1230b of the suspension plate 1230. The brackets 1232 are connected between the suspension plate 1230 and the outer frame 1231, while two ends of the brackets 1232 are connected to the outer frame 1231 and the other end of the brackets 1232 is connected to the suspension plate 1230. The brackets 1232, the suspension plate 1230 and the outer from 1231 collaboratively define a plurality of plate gaps 1235 for allowing the gas to flow through. The configuration, disposition and the number of the suspension plate 1230, the outer frame 1231 and the brackets 1232 are not limited herein and may be varied according to the practical requirements. Moreover, the outer frame 1231 is formed with a conducting pin 1234 protruding outwardly from the outer frame 1231 so as to be electrically connected to the driving power.

In some embodiments, the suspension plate 1230 has a bulge 1230c that makes the suspension plate 1230 a stepped structure. The bulge 1230c is formed on the first surface 1230a of the suspension plate 1230. The bulge 1230c may be a circular protrusion, but no limit thereto. The outer frame 1231 has a first frame surface 1231a and a second frame surface 1231b. Each of the brackets 1232 has a first bracket surface 1232a and a second bracket surface 1232b. Referring to FIGS. 4A to 4C, a surface of the bulge 1230c of the suspension plate 1230 is coplanar with the first frame surface 1231a of the outer frame 1231, and the first surface 1230a of the suspension plate 1230 is coplanar with the first bracket surface 1232a of the bracket 1232. Moreover, a specific depth is formed between the bulge 1230c of the suspension plate 1230 (or the first frame surface 1231a of the outer frame 1231) and the first surface 1230a of the suspension plate 1230 (or the first bracket surface 1232a of the bracket 1232). Referring to FIGS. 4B and 4C. The second surface 1230b of the suspension plate 1230, the second frame surface 1231b of the outer frame 1231 and the second bracket surface 1232b of the bracket 1232 are coplanar with each other. The piezoelectric plate 1233 is attached on the second surface 1230b of the suspension plate 1230. In some embodiments, the suspension plate 1230, the brackets 1232 and the outer frame 1231 is formed as one piece and is made of metal (e.g., a stainless steel plate), but no limit thereto.

Referring back to FIGS. 3A and 3B, in some embodiments, the first insulation plate 1241, the conducting plate 125 and the second insulation plate 1242 of the actuating device 12 are stacked on each other sequentially and located at a side of the piezoelectric actuator 123 that is distal from the resonance plate 122. The shapes of the first insulation plate 1241, the conducting plate 125 and the second insulation plate 1242 substantially match the shape of the outer frame 1231 of the piezoelectric actuator 123. In some embodiments, the first insulation plate 1241 and the second insulation plate 1242 are made of insulating material (e.g. plastic material) for providing insulating efficacy, but no limit thereto. In some embodiments, the conducting plate 125 is made of electrically conductive material (e.g. metallic material) for providing electrically conducting efficacy, but no limit thereto. In some embodiments, the conducting plate 125 is formed with a conducting pin 1251 so as to be electrically connected to the driving power. The conducting pin 1234 of the piezoelectric actuator 123 and the conducting pin 1251 of the conducting plate 152 extend outwardly from the pin opening 112a.

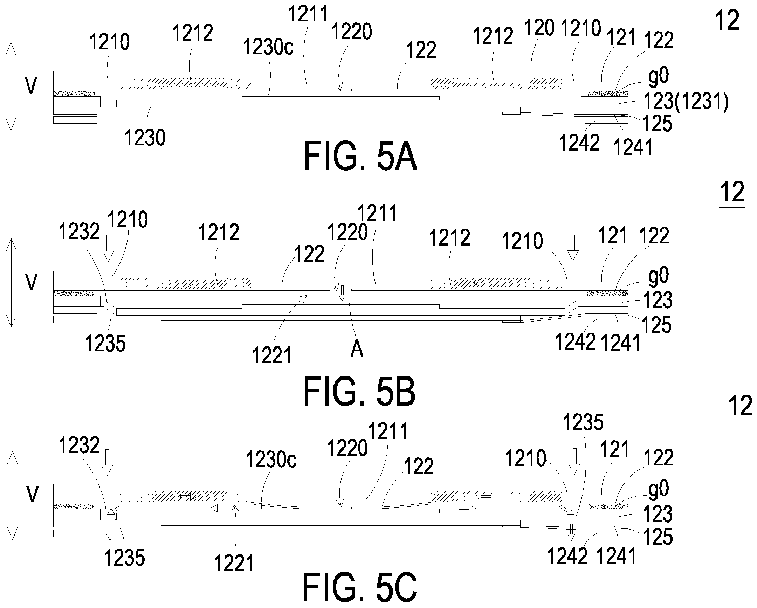

FIGS. 5A to 5E are schematic diagrams illustrating the actuations of the actuating device of the actuating and sensing apparatus. As shown in FIG. 5A, the gas inlet plate 121, the resonance plate 122, the piezoelectric actuator 123, the first insulation plate 1241, the conducting plate 125 and the second insulation plate 1242 are stacked on each other sequentially. Moreover, a gap g0 is formed between the resonance plate 122 and the piezoelectric actuator 123. In some embodiments, the gap g0 between the resonance plate 122 and the outer frame 1231 of the piezoelectric actuator 123 is filled with a filler (e.g. a conductive adhesive), but no limit thereto. In such a manner, a depth between the resonance plate 122 and the bulge 1230c of the suspension plate 1230 of the piezoelectric actuator 123 can be maintained. The gap g0 ensures the proper distance between the resonance plate 122 and the bulge 1230c of the suspension plate 1230 of the piezoelectric actuator 123, so that the gas not only can be transported quickly, but also the contact interference is reduced and the generated noise is largely reduced. In some other embodiments, the thickness of the outer frame 1231 of the piezoelectric actuator 123 is increased, so that a gap can still be formed between the resonance plate 122 and the piezoelectric actuator 123.

Referring to FIGS. 5A to 5E, a convergence chamber A is formed between the central aperture 1220 of the resonance plate 122 and the central cavity 1211 of the gas inlet plate 121 for converging the gas. A third chamber 1221 is formed between the resonance plate 122 and the piezoelectric actuator 123 for temporarily storing the gas. The third chamber 1221 is in communication with the convergence chamber A through the central aperture 1220 of the resonance plate 122. Peripheral regions of the third chamber 1221 is in communication with the compartment 115 of the housing 11 (see FIG. 2) through the plate gaps 1235 between the brackets 1232 of the piezoelectric actuator 123.

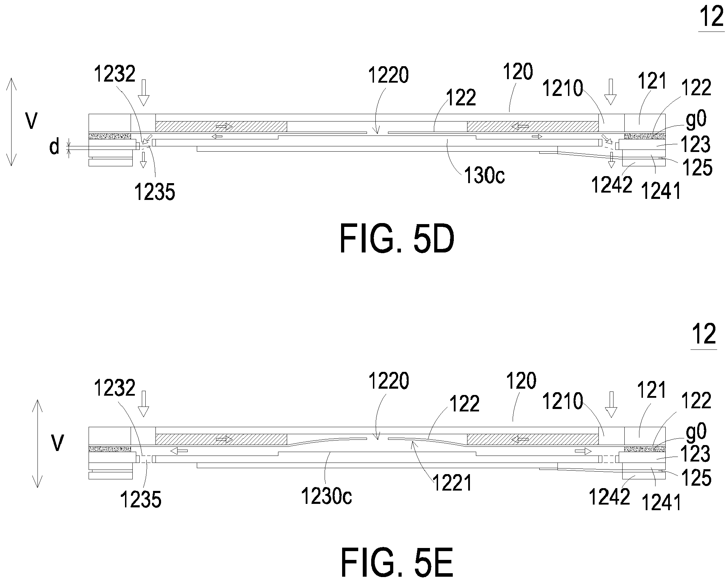

In some embodiment, when the actuating device 12 is driven, the piezoelectric actuator 123 vibrates along the vertical direction V in a reciprocating manner by using the bracket 1232 as a fulcrum. As shown in FIG. 5B, when the piezoelectric actuator 123 first vibrates away from the resonance plate 122 in response to an applied voltage, the gas is inhaled from the at least one gas inlet hole 1210 of the gas inlet plate 121. The gas is then converged to the central cavity 1211 of the gas inlet plate 121 through the at least one convergence channel 1212, and transported into the third chamber 1221 through the central aperture 1220 of the resonance plate 122. As the piezoelectric actuator 123 vibrates, the resonance plate 122 resonates to vibrate in the vertical direction V in a reciprocating manner. As shown in FIG. 5C, the resonance plate 122 vibrates away from the gas inlet plate 121 and attaches on the bulge 1230c of the suspension plate 1230 of the piezoelectric actuator 123. Owing to the deformation of the resonance plate 122 described above, a middle communication space between the third chamber 1221 and the central cavity 1211 is sealed so as to compress the volume of the third chamber 1221. Under this circumstance, the pressure gradient occurs to push the gas in the third chamber 1221 toward the peripheral regions of the third chamber 1221, and the gas is then further transported through the plate gaps 1235 of the piezoelectric actuator 123. As shown in FIG. 5D, the resonance plate 122 returns to its original position when the piezoelectric actuator 123 deforms toward the resonance plate 122 during the reciprocating vibration. Consequently, the volume of the third chamber 1221 is continuously compressed. Since the piezoelectric actuator 123 moves toward the resonance plate 122 for a displacement d, the gas is continuously pushed toward the peripheral regions of the third chamber 1221. Meanwhile, the gas is continuously inhaled from the at least one gas inlet hole 1210 of the gas inlet plate 121 and transported to the central cavity 1211. Subsequently, as shown in FIG. 5E, the resonance plate 122 moves toward the gas inlet plate 121 resonating to the vibration of the piezoelectric actuator 123. Under this circumstance, the gas in the central cavity 1211 is transported to the third chamber 1221 through the central aperture 1220 of the resonance plate 122, and then the gas is transported to be discharged out of the actuating device 12 through the plate gaps 1235 of the piezoelectric actuator 123. In such a manner, the pressure gradient is generated in fluid channels of the actuating device 12 to facilitate the gas to flow at a high speed. Moreover, since there is an impedance difference between the inhale direction and the discharge direction, the gas can be transported from the inhale end to the discharge end. In addition, even if a gas pressure exists at the discharge end, the actuating device 12 still has the capability of discharging the gas outwardly while achieving the silent efficacy. In some embodiments, the vibration frequency of the resonance plate 122 in the vertical direction V in the reciprocating manner is identical to the vibration frequency of the piezoelectric actuator 123. That is, the resonance plate 122 and the piezoelectric actuator 123 may synchronously vibrate in a same direction. It is noted that, numerous modifications and alterations of the actions of the actuating device 12 may be made while retaining the teachings of the disclosure.

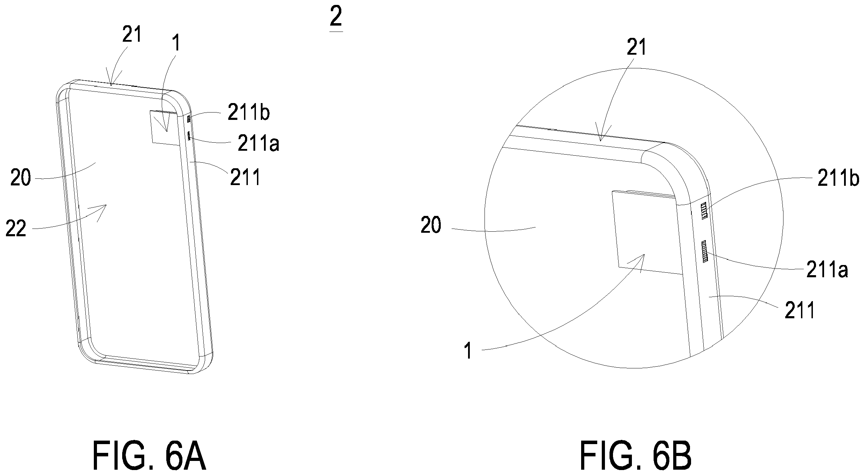

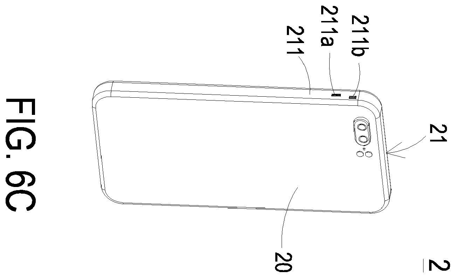

FIG. 6A is a schematic perspective view illustrating the actuating and sensing apparatus and a case of an electronic device according to some embodiments of the present disclosure. FIG. 6B is a partially enlarged schematic view of the actuating and sensing apparatus and the case of the electronic device according to some embodiments of the present disclosure. FIG. 6C is another schematic perspective view illustrating the case of the electronic device according to the embodiment of the present disclosure. The actuating and sensing apparatus 1 of the present disclosure may be applied to a case 2 of any portable electronic device. For example, a notebook, a smart phone, a smart band, a tablet or any other appropriate portable electronic device. In some embodiments, the case 2 for the smart phone is exemplified, but not limited thereto. As shown in FIG. 6A, the case 2 has a plate 20 and lateral wall 21. The lateral wall 21 extends from the plate 20 and has a first wall section 211. The first wall section 211 of the lateral wall 21 is formed with an inlet 211a and an outlet 211b. An accommodating space 22 of the case 2 is defined by the lateral wall 21 and the plate 20 collaboratively. In some embodiments, the actuating and sensing apparatus 1 is disposed within the accommodating space 22, and is mounted on the plate 20. The inhale aperture 111a and the discharge aperture 111b of the actuating and sensing apparatus 1 are disposed corresponding in position to the inlet 211a and the outlet 211b of the first wall section 211 of the lateral wall 21, respectively. Referring to FIGS. 2 and 6B, when the actuating device 12 of the actuating and sensing apparatus 1 is driven, the gas within the compartment 115 of the actuating and sensing apparatus 1 is guided to the exterior of the case 2 through the discharge aperture 111b of the housing 11 and the outlet 211b of the first wall section 211 in sequence, and the pressure gradient is generated in the compartment 115 so as to introduce the gas from the exterior of the case 2 into the compartment 115 through the inlet 211a and the inhale aperture 111a to make the gas measured by the sensor 13. Consequently, the air circulation is formed. Since the actuating and sensing apparatus 1 is mounted within the case 2 of the smart phone or any other portable electronic device, the gas could be inhaled and discharged through the same side wall, for example, the first wall section 211 of the lateral wall 21 of the casing 2. Moreover, since the overall volume and thickness of the actuating and sensing apparatus 1 are reduced, the actuating and sensing apparatus 1 can be applied to the smart phone or the portable electronic device. By using the portable electronic device of the present disclosure, the information relating to the environmental gas can be immediately measured everywhere and at any time. If the concentration or content of a harmful gas in the environmental air exceeds a threshold value, the actuating and sensing apparatus 1 reminds the user to take the protective actions immediately for preventing gas poisoning and gas explosion.

From the above descriptions, the present disclosure provides the actuating and sensing apparatus. The environmental gas is introduced into the compartment through the inhale aperture of the first side wall of the housing for being measured by the sensor. When the piezoelectric actuator is driven, the pressure gradient is generated in the fluid channels and the chambers of the actuating device to facilitate the gas to flow out from the discharge aperture of the first side wall of the housing at a high speed. Consequently, the gas can be circulated and quickly transported while achieving silent efficacy, and the gas could be inhaled and discharged through the same side wall. Due to the disposition of the first protective film and the second protective film, the components within the housing won't be rusted or damaged by the moisture or the dust. Consequently, the gas transportation efficiency is enhanced. Since the possibility of causing the damage to the actuating and sensing apparatus is reduced, the performance of the gas transportation and the gas sensing will be enhanced. Moreover, since the overall volume and thickness of the actuating and sensing apparatus are reduced, and the actuating and sensing apparatus has the feature that the gas could be inhaled and discharged through the same side wall, the feather is beneficial for applying the actuating and sensing apparatus to a miniature device or the portable electronic device. By using the portable electronic device of the present disclosure, the information relating to the environmental gas can be immediately measured everywhere and at any time. If the concentration or content of a harmful gas in the environmental air exceeds a threshold value, the actuating and sensing apparatus reminds the user that the concentration or content of the harmful gas in the environment is too high. After realizing the harmful level of the environmental gas, the user is given a chance to escape quickly or take protective actions, instantly.

While the disclosure has been described in terms of what is presently considered to be the most practical and preferred embodiments, it is to be understood that the disclosure needs not be limited to the disclosed embodiment. On the contrary, it is intended to housing various modifications and similar arrangements included within the spirit and scope of the appended claims which are to be accorded with the broadest interpretation so as to encompass all such modifications and similar structures.

## Claims

1. An actuating and sensing apparatus, comprising:
a circuit board (10);
a housing (11) disposed on the circuit board (10) and having a compartment (115), wherein the compartment (115) is in communication with the exterior of the housing (11) through an inhale aperture (111a) and a discharge aperture (111b), which are both disposed on a first side wall (111);
an actuating device (12) disposed within the compartment (115); and
a sensor (13) disposed within the compartment (115);
wherein when the actuating device (12) is driven, gas within the compartment (115) is guided to the exterior of the housing (11) through the discharge aperture (111b), and a pressure gradient is generated in the compartment (115) so as to introduce the gas from the exterior of the housing (11) into the compartment (115) through the inhale aperture (111a) to make the gas measured by the sensor (13).

2. The actuating and sensing apparatus according to claim 1, wherein the sensor (13) is disposed corresponding in position to the inhale aperture (111a) of the housing (11).

3. The actuating and sensing apparatus according to claim 1, wherein the sensor (13) is disposed corresponding in position to the discharge aperture (111b) of the housing (11).

4. The actuating and sensing apparatus according to claim 1, further comprising a first protective film (141) and a second protective film (142), the first protective film (141) and the second protective film (142) are respectively and closely disposed at the inhale aperture (111a) and the discharge aperture (111b), wherein the first protective film (141) and the second protective film (142) are waterproof and dustproof film structures, which allow the gas to pass therethrough.

5. The actuating and sensing apparatus according to claim 4, wherein the first protective film (141) and the second protective film (142) comply with IP64 waterproof and dustproof rating of International Protection Marking.

6. The actuating and sensing apparatus according to claim 4, wherein the first protective film (141) and the second protective film (142) comply with IP68 waterproof and dustproof rating of International Protection Marking.

7. The actuating and sensing apparatus according to claim 1, wherein the actuating device includes:
a gas inlet plate (121) having at least one gas inlet hole (1210);
a resonance plate (122); and
a piezoelectric actuator (123),
wherein the gas inlet plate (121), the resonance plate (122) and the piezoelectric actuator (123) are stacked on each other sequentially, and a gap is formed between the resonance plate (122) and the piezoelectric actuator (123) and is defined as a third chamber (1221), when the piezoelectric actuator (123) is driven, the gas is inhaled into the third chamber (1221) through the at least one gas inlet hole (1210) of the gas inlet plate (121) and the resonance plate (122) so as to introduce the gas from the inhale aperture (111a).

8. The actuating and sensing apparatus according to claim 7:
wherein the gas inlet plate (121) further has at least one convergence channel (1212) and a central cavity (1211);
wherein the at least one convergence channel (1212) is aligned with the at least one gas inlet hole (1210), and the gas inhaled into the at least one gas inlet hole (1210) is guided to the central cavity (1211);
wherein the resonance plate (122) has a central aperture (1220) corresponding in position to the central cavity (1211) of the gas inlet plate (121);
wherein the piezoelectric actuator (123) has a suspension plate (1230), an outer frame (1231), at least one bracket (1232) and a piezoelectric plate (1233); and
wherein the at least one bracket (1232) is connected between the suspension plate (1230) and the outer frame (1231), and the piezoelectric plate (1233) is attached on a surface (1230b) of the suspension plate (1230).

9. The actuating and sensing apparatus according to claim 7:
wherein the actuating device (12) further includes at least one insulation plate (1241, 1242) and a conducting plate (125); and
wherein the at least one insulation plate (1241, 1242) and the conducting plate (125) are stacked on each other sequentially and located at a side of the piezoelectric actuator (123) that is distal from the resonance plate (122).

10. The actuating and sensing apparatus according to claim 1, wherein the sensor (13) is configured as at least one selected from the group consisting of an oxygen sensor, a carbon monoxide sensor, a carbon dioxide sensor, a temperature sensor, an ozone sensor, a sulfur dioxide sensor, a nitrogen dioxide sensor, a volatile organic compound sensor and a combination thereof.

11. A case for using in an electronic device, the case comprising:
an actuating and sensing apparatus (1) including a circuit board (10), a housing (11), an actuating device (12) and a sensor (13), wherein the housing (11) is disposed on the circuit board (10) and has a compartment (115), the compartment (115) is in communication with the exterior of the housing (11) through an inhale aperture (111a) and a discharge aperture (111b), which are disposed on a first side wall (111), the actuating device (12) and the sensor (13) are disposed within the compartment (115);
a plate (20); and
a lateral wall (21) extending from the plate (20) and having a first wall section (211), an inlet (211a) and an outlet (211b) are formed in the first wall section (211);
wherein the actuating and sensing apparatus (1) are disposed on the plate (20), and the inhale aperture (111a) and the discharge aperture (111b) of the actuating and sensing apparatus (1) are disposed corresponding in position to the inlet (211a) and the outlet (211b) of the first wall section (211), respectively, when the actuating device (12) of the actuating and sensing apparatus (1) is driven, gas within the compartment (115) is guided to the exterior of the case (2) through the discharge aperture (111b) and the outlet (211b) in sequence, and a pressure gradient is generated in the compartment (115) so as to introduce the gas from the exterior of the case (2) into the compartment (115) through the inlet (211a) and the inhale aperture (111a) in sequence to make the gas measured by the sensor (13).

12. The case according to claim 11, wherein the sensor (13) of the actuating and sensing apparatus (1) is disposed corresponding in position to the inhale aperture (111a) of the housing (11).

13. The case according to claim 11, wherein the sensor (13) of the actuating and sensing apparatus (1) is disposed corresponding in position to the discharge aperture (111b) of the housing (11).

14. The case according to claim 11, wherein the actuating and sensing apparatus (1) further includes a first protective film (141) and a second protective film (142), the first protective film (141) and the second protective film (142) are respectively and closely disposed at the inhale aperture (111a) and the discharge aperture (111b), wherein the first protective film (141) and the second protective film (142) are waterproof and dustproof film structures, which allow the gas to pass therethrough.

15. An actuating and sensing apparatus, comprising:
at least one circuit board (10);
at least one housing (11) disposed on the circuit board (10) and having at least one compartment (115), wherein the compartment (115) is in communication with the exterior of the housing (11) through at least one inhale aperture (111a) and at least one discharge aperture (111b), which are disposed on a first side wall (111);
at least one actuating device (12) disposed within the compartment (115); and
at least one sensor (13) disposed within the compartment (115);
wherein when the actuating device (12) is driven, gas within the compartment (115) is guided to the exterior of the housing (11) through the discharge aperture (111b), and a pressure gradient is generated in the compartment (115) so as to introduce the gas from the exterior of the housing (11) into the compartment (115) through the inhale aperture (111a) to make the gas measured by the sensor (13).
